# EUROPÄISCHE PATENTSCHRIFT

(11) **EP 0 746 624 B1**
(45) Veröffentlichungstag und Bekanntmachung des Hinweises auf die Patenterteilung: **19.12.2001**
(21) Anmeldenummer: 95911218.6
(22) Anmeldetag: 28.02.1995
(51) Int. Cl.: C12N 15/86, C12N 7/01, A61K 48/00

(54) **VEKTOR FÜR DIE LEBERSPEZIFISCHE GENEXPRESSION**
VECTOR FOR LIVER-SPECIFIC GENE EXPRESSION
VECTEUR POUR L'EXPRESSION SPECIFIQUE DU FOIE

(30) Priorität: 04.03.1994 DE 4407859
(43) Veröffentlichungstag der Anmeldung: 11.12.1996
(73) Patentinhaber: Max-Planck-Gesellschaft zur Förderung der Wissenschaften e.V., 80539 München (DE)
(72) Erfinder: HOFMANN, Christian, D-13125 Berlin (DE); SANDIG, Volker, D-13125 Berlin (DE); STRAUSS, Michael, D-13187 Berlin (DE)
(74) Vertreter: Weiss, Wolfgang, Dipl.-Chem. Dr.
(86) Internationale Anmeldenummer: DE9500333
(87) Internationale Veröffentlichungsnummer: WO9523866

(56) Entgegenhaltungen:
- WO-A-88/10304
- WO-A-91/02056
- DE-C- 4 339 922
- US-A- 5 004 687
- PROC. NATL.ACAD SCI., Bd. 90, Nr. 6, 15.März 1993 NATL. ACAD SCI.,WASHINGTON,DC,US;, Seiten 2122-2126, R.J. CHRISTIANO ET AL. 'Hepatic gene therapy: Adenovirus enhancement of receptor-mediated gene delivery and expression in primary hepatocytes'
- PROC. NATL.ACAD SCI., Bd. 88, Nr. 10, 15.Mai 1991 NATL. ACAD SCI.,WASHINGTON,DC,US;, Seiten 4513-4517, R. HOOPES AND G.R. ROHRMANN 'In vitro transcription of baculovirus immediate early genes: Accurate mRNA initiation by nuclear extracts from both insect and human cells'
- J. GENERAL VIROLOGY, Bd. 73, Nr. 7, Juli 1992 READING, BERKS, GB, Seiten 1877-1880, H. MORI ET AL. 'Foreign gene expression by a baculovirus vector with an expanded host range'

## Beschreibung

Die Erfindung betrifft einen Vektor für die leberspezifische Gentherapie; Anwendungsgebiete sind die Medizin und die Gentechnik.

In den vergangenen Jahren sind zahlreiche Methoden und Vektoren für die Gentherapie entwickelt worden (Übersicht in Mulligan/1993/Science 260, 926). Dabei werden viele Vektoren, vor allem solche, die von Retroviren oder Adenoviren abgeleitet sind, favorisiert. Beide Virus-Vektortypen sind relativ breit anwendbar, wobei retrovirale Vektoren nur bei teilungsfähigen Zellen effektiv sind und Adenoviren auch bei ruhenden Zellen funktionieren. Beide Vektortypen sind zwar für die Genübertragung in Leberzellen (Hepatozyten) in vitro geeignet, können aber für eine in vivo Anwendung zur Gentherapie beim Menschen kaum in Betracht gezogen werden. Während für die Anwendung retroviraler Vektoren eine Leberteilresektion zur Stimulierung von Zellteilung (Regeneration) erforderlich wird, ist der adenovirale Gentransfer nicht sehr stabil (keine Integration in das Genom).
Alternative Vektoren mit potentieller Anwendbarkeit für den Lebergentransfer basieren auf Liposomen oder auch auf Multikomponenten-Partikeln mit Proteindomänen, die spezifisch an bestimmte Rezeptoren der Leber (z.B. Asialoglykoprotein-Rezeptor) binden und durch deren Internalisierung in die Zelle aufgenommen werden können (Übersicht in: Versland et al/1992/ Seminars in Liver Disease 12, 332). Ein wesentlicher Nachteil dieser Vektoren besteht in der Aufnahme über den endozytotischen Weg der zu einer Degration eines großen Teils der Vektoren und ihrer DNA in den Endosomen führt, so daß nur wenig funktionsfähige DNA den Zellkern erreichen kann.
Eine Lösung für dieses Problem wurde zwar für die in vitro Anwendung gefunden; diese ist aber nicht auf die Situation in vivo (am Patienten) anwendbar. Sie basiert auf der gleichzeitigen Infektion der Zielzellen mit Adenovirus, was zur Auflösung der Endosomen und Freisetzung von Vektor (DNA) führt (Curiel, D.T., Agrawal, S., Wagner, E. und Cotten, M./1991, PNAS 88, 8850-8854).
In P 43 39 922.3 wurde vorgeschlagen, ein an einen Promoter gekoppeltes therapeutisches Gen, das bei der zu behandelnden Krankheit defekt ist, mit einer Peptidhülle zu verpacken und an Komponenten des Hepatitis B-Virus zu koppeln. Damit wurde eine wichtige Voraussetzung für eine Therapie genetischer Erkrankungen der Leber geschaffen.

Ziel der Erfindung ist die Konstruktion eine Vektors, der Leberzellen in vivo hochspezifisch findet, von den Zellen effektiv aufgenommen wird und auch mehrere bzw. sehr große therapeutische Gene aufnehmen kann. Der Vektor soll für den Gentherapie beim Menschen einsetzbar sein.

Die Erfindung wird gemäß den Ansprüchen 1, 9 und 10 realisiert, die Unteransprüche sind Vorzugsvarianten.
Der wesentliche Bestandteil des erfindungsgemäßen Vektors ist ein Insektenvirus, vorzugsweise ein Vertreter der Baculoviren oder ein nukleares Polyhedrosis-Virus. Dieses Insektenvirus enthält
- eine oder mehrere therapeutische DNA-Sequenzen
- einen für die Genexpression in der Leber geeigneten Promoter
- und ggf. eine Etablierungssequenz.
Die bevorzugt eingesetzten Baculoviren gehören zu einer Familie großer DNA-Viren, deren Wirtsspektrum natürlicherweise ausschließlich auf Arthropoden beschränkt ist. Ihr Genom (80kbp-200kpb) ist in flexible Nukleokapside verpackt, die die Insertion großer Mengen Fremd-DNA ermöglicht. Die ausgesprochene Wirtsspezifität der Baculoviren nahm man als Grundlage für ihren Einsatz in der biologischen Schädlingsbekämpfung, als man erkannte,daß durch den Einsatz chemischer Insektizide auch potentielle Gefahren für Mensch und Umwelt hervorgerufen werden können.
Eine Voraussetzung für diesen Einsatz besteht darin, daß eine Expression von Baculovirusgenen in menschlichen Zellen nicht stattfindet. In der Tat wurde bei Untersuchungen an zahlreichen humanen Zelltypen festgestellt, daß bei der Behandlung mit Baculoviren keine signifikanten Infektionen eintreten.

Als therapeutische DNA-Sequenz für den erfindungsgemäßen Vektor wird die cDNA eines Gens verwendet, das bei der zu behandelnden Krankheit defekt ist, d.h. fehlt oder durch Mutation verändert ist. Beispiele für solche Gene sind das LDL-Rezeptor-Gen, das Gen für das Alpha-1-Antitrypsin, für die Blutgerinnungsfaktoren VIII und IX, für Erythropoetin oder für Thymidinkinase. Man kann auch einen Teil einer genomischen Sequenz einsetzen, die eine Mutation im Zielgen überspannt und mit dieser homolog rekombinieren kann.

Als Promotoren dienen in erster Linie starke virale Promotoren, vorzugsweise der sehr frühe Promoter des Cytomegalievirus (CMV). Ebenfalls in Betracht kommen leberspezifische Promotoren, bevorzugt Promoter/Enhancer des Hepatitis B-Virus wie z.B. die Kombination core-Promoter/enhancer II. Sie sind neben ihrer Spezifität auch klein genug, um leicht in einen Expressionsvektor eingebaut werden zu können. Auch in Betracht für die Konstruktion des erfindungsgemäßen Vektors kommen Promotoren leberspezifischer Gene wie Albumin, PEPCK (Phosphoenolpyruvatcarboxykinase) oder OTC (Ornithintranscarbamylase).

Die Etablierungssequenz hat die Aufgabe, für eine Stabilisierung des Vektors in der Zelle ohne Integration in das Genom zu sorgen. Sie wird besonders in den Fällen verwendet, wenn eine Langzeitexpression erforderlich ist. Bevorzugte Etablierungssequenzen gemäß der Erfindung sind virale Kernetablierungssequenzen, wie die des Epstein-Barr-Virus, oder autonome Replikationssequenzen aus dem Säugergenom.

Die Herstellung der neuen Vektoren erfolgt in den folgenden wesentlichen Schritten
- Klonierung der therapeutischen DNA-Sequenz zusammen mit dem Promoter in einem Rekombinationsvektor
- ggf. Einfügung einer Etablierungssequenz vor oder nach der Klonierung
- Transfektion des erhaltenen Konstrukts gemeinsam mit der DNA eines Insektenvirus in Insektenzellen und
- Gewinnung des in den Insektenzellen verpackten Vektors aus dem Überstand der Insektenzellkultur.

Durch die Erfindung wird ein neuartiger Vektor für den leberspezifischen Gentransfer geschaffen, der gegenüber den bisher entwickelten Virusvektoren auf der Basis von Retroviren oder Adenoviren erhebliche Vorteile bietet. Dazu gehören die Leberspezifität, die fast unbegrenzte Möglichkeit, Fremd-DNA einzubauen, die Infektion nicht teilungsfähiger Zellen, die fehlende Cytotoxität und die einfache Generierung hochtitriger rekombinanter Viren.
Es ist besonders überraschend, daß der Vektor hochspezifisch Hepatozyten ihfiziert. Er ermöglicht, ein gewünschtes Gen in die Leber eines Patienten einzuführen und seinen Weg zum Funktionsort optimal zu gestalten. Das erfolgt beispielsweise dadurch, daß der Vektor konfektioniert und einem Patienten über die Blutbahn, vorzugsweise über die Portalvene der Leber, verabreicht wird. Dadurch wird eine wesentliche Voraussetzung für eine Therapie genetischer Erkrankungen der Leber geschaffen.

Die Erfindung soll nachfolgend durch ein Ausführungsbeispiel näher erläutert werden.

### Ausführungsbeispiel

### Baculovirusvektor für Luziferaseexpression in Hepatozyten

### 1. Konstruktion des Baculovirustransfervektor

1.1. Das *Pvu*II-Fragment des pCMV-Luci (Müller et al., Proc. Natl. Acad. Sci. U.S.A. 91, 2945 (1994)) enthält den Cytomegalieviruspromoter, das Luciferasereportergen und das CMV-Polyadenyllerungsslgnal als Expressionskassette. Es wird in die *Sma*I Schnittstelle des pVL 1392 kloniert. Das über eine Quiagensäule gereinigte Plasmid (pVL-CMV-Luci) wird für die Herstellung eines rekombinanten Baculovirus verwendet.
1.2. Durch *Bam*HI-*Hind* III-Restriktion und Endenauffüllung durch Klenow-Enzym aus dem Plasmid T7-lol (Dissertation A. Lieber und V. Sandig) erhält man das Luciferasegen. Nach Klonierung in die *Sma*I-Schnittstelle des pVL-1392 steht es unter der Kontrolle des Polyhedrinpromotors (pVL-PP-Luci). Dieses Konstrukt dient als Negativkontrolle, um den Einfluß des Säugerpromotors bei der Hepatozyteninfektion zu verdeutlichen.
1.3. Das Luciferasegen wird in analogen Konstrukten durch therapeutische Gene, wie z.B. das LDL-Rezeptorgen ersetzt. Anstelle des CMV-Promotor kann ein leberspezifischer Säugerpromotor (Hepatitis) verwendet werden. Die die Expressionskassette flankierenden Polyhedrinsequenzen können auch durch andere nicht essentielle Baculovirussequenzen (z.B. p94) ausgetauscht werden.

### 2. Herstellung rekombinanter Viren

3 µg pVL-CMV-Luci bzw. pVL-PP-Luci und je 0,5 µg Baculovirus-Wildtyp-DNA (Baculo-Gold, PharMingen) werden mittels Lipofektin (BRL) in 2 Millionen Spodoptera frugiperda-Insektenzellen (Sf9, ATCC) kotransfiziert. Durch homologe Rekombination, vermittelt durch die Polyhedrin-Sequenzen der Transfervektoren, entstehen rekombinante Baculoviren (Bac-CMV-Luci, Bac-PP-Luci), die das Luciferase- Gen unter Kontrolle des CMV- bzw. Polyhedrinpromotors tragen.

### 3. Vermehrung und Reinigung der rekombinanten Baculoviren

Sf9-Insektenzellen werden in Flaschen- oder Spinnerkultur mit einer MOI von 1 drei Tage lang mit dem Bac-CMV-Luci bzw. Bac-PP-Luci infiziert. Die jeweils entstehenden Viren werden durch Zentrifugation von den Insektenzellen abgetrennt und anschließend pelletiert (12000g, 45 min). Durch Saccharosedichtegradienten-Zentrifugation (Gradient 24-62%) erhält man nach 4h bei 100000g eine Bande gereinigten Virus, die durch zweimaliges Pelletieren in den Applikationspuffer (isotonische Kochsalzlösung, resp. Zellkulturmedium) überführt wird.

### 4. Versuche zur Spezifität der Infektion bzw. Luziferaseexpression des Bac-CMV-Luci in Leberzellen

Um die Zelispezifität der Baculovirusinfektion zu untersuchen, werden Säugerzellinien verschiedenster Gewebe und Spezies und primäre humane (phH) sowie primäre murine (pmH) Hepatozyten mit Bac-CMV-Luci bzw. Bac-PP-Luci infiziert (MOI 100). Nach 1,5 Tagen wird die Luciferaseexpression gemessen. Das Ergebnis der Luziferaseexpressionswerte zeigt die Spezifität des Bac-CMV-Luci in Bezug auf die Hepatozyteninfektion. Das Luziferasegen unter dem Polyhedrinpromotor (b: Bac-PP-Luci Infektion verschiedener Zellinien) ist ausschließlich in Insektenzellen aktiv.

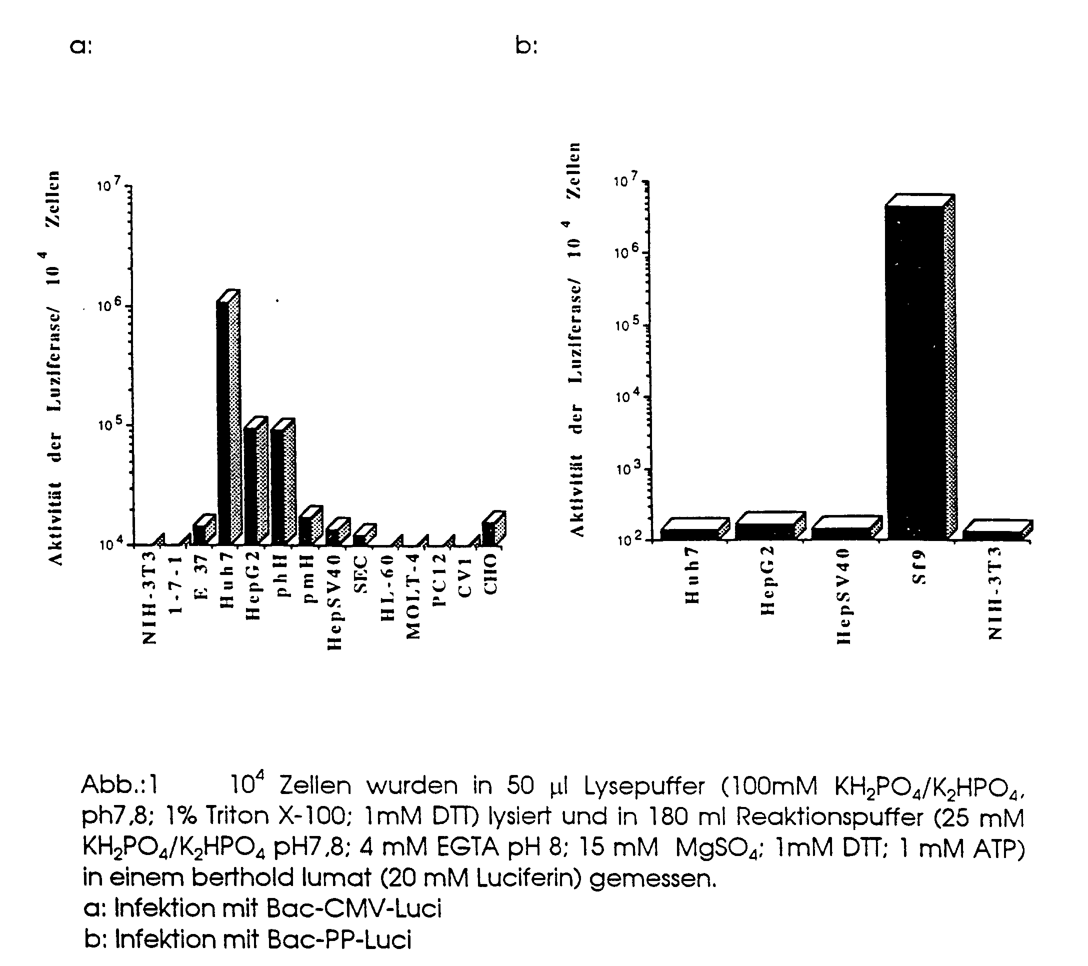
a:

b:

Aktivität der Luziferase/ 10 4 Zellen

10 7

10 6

10 5

10 4

NIH-3T3
1-7-1
E 37
Huh7
HepG2
phH
pmH
HepSV40
SEC
HL-60
MOLT-4
PC12
CV1
CHO


Aktivität der Luziferase/ 10 4 Zellen

10 7

10 6

10 5

10 4

10 3

10 2

Huh7
HepG2
HepSV40
Sf9
NIH-3T3


Abb.:1 10⁴ Zellen wurden in 50 µl Lysepuffer (100mM KH₂PO₄/K₂HPO₄,
ph7,8; 1% Triton X-100; 1mM DTT) lysiert und in 180 ml Reaktionspuffer (25 mM
KH₂PO₄/K₂HPO₄ pH7,8; 4 mM EGTA pH 8; 15 mM MgSO₄; 1mM DTT; 1 mM ATP)
in einem berthold lumat (20 mM Luciferin) gemessen.
a: Infektion mit Bac-CMV-Luci
b: Infektion mit Bac-PP-Luci

### 5. Hinweise auf eine Proteinrezeptorvermittelte Endozytose des Baculovirus in Hepatozyten (Huh7):

Proteasebehandlung der Hepatozyten verringern die Proteinrezeptorzahl, so daß eine Reduktion der Luziferaseaktivität nach Bac-CMV-Luci Infektion eintritt:

| Enzyme | Konzentration | % Reduktion der Luziferaseaktivität |
|---|---|---|
| Trypsin | 2mg/ml | 50.4 |
| Papain | 1mg/ml | 36.9 |
| Pronase E | 1mg/ml | 52.6 |
| Abb.:2 Huh7-Zellen wurden 15 min mit den angegebenen Proteasen behandelt und nach deren Inaktivierung mit Bac-CMV-Luci (MOI100) 1h lang infiziert. Analog wurden Huh7-Zellen 3h nach der 1 stündigen Virusinfektion behandelt (Referenzwert 100%). Die Reduktion der Luziferaseaktivität (Messung nach 1,5 d) ergibt sich aus dem Luziferasewert der vorher proteasebehandelten Hepatozyten zu dem Referenzwert. | | |

Chloroquin und Colchizin greifen in den Endozytoseweg des Virus bzw. seiner Wanderung in den Hepatozytenkern ein. Die Applikation beider Chemikalien verringert ebenfall die Luziferaseaktivität.

| | Konzentration | % Reduktion der Luziferaseaktivität |
|---|---|---|
| Chloroquin | 0.5 mM | 100 |
| | 0.1 mM | 98.4 |
| | 0.03 mM | 92 |
| | 0.01 mM | 81 |
| Colchizin | 18 mg/ml | 69.4 |
| | 6 mg/ml | 56 |
| | 2 mg/ml | 55 |
| Abb.:3 Huh7-Zellen wurden mit Bac-CMV-Luci (MOI 100) bei gleichzeitige Zugabe von Chloroquin bzw. Colchizin infiziert. Die Reduktion der Luziferaseaktivität bezieht sich auf einen Referenzwert, bei dem die beiden Chemikalien jeweils 12 h nach Infektion gegeben wurden. Die Expression der Luziferase wurde nach 1,5 Tagen gemessen. | | |

## Patentansprüche

1. Vektor geeignet für die leberspezifische Genexpression bestehend aus einem Insektenvirus, welches die Komponenten
- eine Fremd-DNA-Sequenz unter der Kontrolle
- eines für die Genexpression in Leberzellen geeigneten Promotors enthält.

2. Vektor nach Anspruch 1,
**dadurch gekennzeichnet,**
**dass** er eine Etablierungssequenz enthält.

3. Vektor nach Anspruch 1,
**dadurch gekennzeichnet,**
**dass** als Fremd-DNA-Sequenz eine therapeutische Sequenz verwendet wird.

4. Vektor nach einem der Ansprüche 1 bis 3,
**dadurch gekennzeichnet,**
**dass** als Insektenvirus Baculovirus verwendet wird.

5. Vektor nach einem der Ansprüche 1 bis 4,
**dadurch gekennzeichnet,**
**dass** als Insektenvirus nukleares Polyhedrosis-Virus verwendet wird.

6. Vektor nach Anspruch 1 bis 5,
**dadurch gekennzeichnet,**
**dass** als Fremd-DNA-Sequenz die cDNA eines Gens verwendet wird, das bei der zu behandelnden Krankheit defekt ist.

7. Vektor nach einem der Ansprüche 1 bis 6,
**dadurch gekennzeichnet,**
**dass** als Fremd-DNA-Sequenz ein Teil einer genomischen Sequenz, die eine Mutation im Zielgen überspannt und mit dieser homolog rekombinieren kann, verwendet wird.

8. Vektor nach einem der Ansprüche 1 bis 7,
**dadurch gekennzeichnet,**
**dass** als Fremd-DNA-sequenz die cDNA für den LDL-Rezeptor, für alpha-1-Antitrypsin, für die Blutgerinnungsfaktoren VIII und IX, für Erythropoietin oder für Thymidinkinase verwendet wird.

9. Vektor nach einem der Ansprüche 1 bis 8,
**dadurch gekennzeichnet,**
**dass** als Promotor ein starker viraler, vorzugsweise der sehr frühe Promotor des Cytomegalievirus (CMV) oder ein leberspezifischer Promotor, vorzugsweise der des Hepatitis B-Virus, oder ein leberspezifischer Säugerpromotor, vorzugsweise der des Albumingens, verwendet wird.

10. Vektor nach einem der Ansprüche 2 bis 9,
**dadurch gekennzeichnet,**
**dass** als Etablierungssequenz eine virale Kernetablierungssequenz, wie die des Epstein-Barr-Virus, oder eine autonome Replikationssequenz verwendet wird.

11. Rekombinationsvektor zur Herstellung eines rekombinanten Insektenvirusvektors nach einem der Ansprüche 1 bis 10 umfassend eine Fremd-DNA-Sequenz unter Kontrolle eines für die Genexpression in der Leber geeigneten Promotors und Rekombinationssequenzen aus nicht essenziellen Bereichen der Insektenvirus-DNA.

12. Verfahren zur Herstellung des Vektors nach einem der Ansprüche 1 bis 10,
**dadurch gekennzeichnet,**
**dass** die Fremd-DNA-Sequenz zusammen mit dem Promotor in einen Rekombinationsvektor kloniert wird, das erhaltene Konstrukt gemeinsam mit der DNA eines Insektenvirus in Insektenzellen transfiziert und der in den Insektenzellen verpackte Vektor aus dem Überstand der Insektenzellkultur gewonnen wird.

13. Verfahren zur Herstellung des Vektors nach Anspruch 12,
**dadurch gekennzeichnet,**
**dass** eine Etablierungssequenz vor oder nach Klonierung eingefügt wird.

14. Pharmazeutische Zusammensetzung,
**dadurch gekennzeichnet,**
**dass** sie einen rekombinanten Insektenvirusvektor nach einem der Ansprüche 1 bis 10 enthält.

15. Verwendung eines rekombinanten Insektenvirusvektors nach einem der Ansprüche 1 bis 10 oder einer pharmazeutischen Zusammensetzung nach Anspruch 14 zur Herstellung eines Mittels für die leberspezifische Gentherapie.

16. Verwendung nach Anspruch 15,
**dadurch gekennzeichnet,**
**dass** der Vektor in eine Form konfektionert wird, dass er einem Patienten über die Blutbahn, vorzugsweise über die Portalvene der Leber, verabreicht werden kann.

17. Verwendung nach Anspruch 15,
**dadurch gekennzeichnet,**
**dass** der Vektor einem Patienten über den Magen-Darm-Trakt verabreicht werden kann.

## Claims

1. Vector suitable for liver-specific gene expression comprising an insect virus which contains the components
- a foreign DNA sequence under the control
- of a suitable promoter for gene expression in liver cells.

2. Vector as claimed in claim 1,
**wherein**
it contains an establishing sequence.

3. Vector as claimed in claim 1,
**wherein**
a therapeutic sequence is used as the foreign DNA sequence.

4. Vector as claimed in one of the claims 1 to 3,
**wherein**
a baculovirus is used as the insect virus.

5. Vector as claimed in one of the claims 1 to 4,
**wherein**
a nuclear polyhedrosis virus is used as the insect virus.

6. Vector as claimed in claims 1 to 5,
**wherein**
the cDNA of a gene which is defective in the disease to be treated is used as the foreign DNA sequence.

7. Vector as claimed in one of the claims 1 to 7,
**wherein**
a part of a genomic sequence which spans a mutation in the target gene and can homologously recombine with this mutation is used as the foreign DNA sequence.

8. Vector as claimed in one of the claims 1 to 7,
**wherein**
the cDNA for the LDL receptor, for alpha-1-antitrypsin, for the blood coagulation factors VIII and IX, for erythropoietin or for thymidine kinase is used as the foreign DNA sequence.

9. Vector as claimed in one of the claims 1 to 8,
**wherein**
a strong viral promoter is used as the promoter and preferably the very early promoter of the cytomegalo-virus (CMV) or a liver-specific promoter preferably that of the hepatitis B virus or a liver-specific mammalian promoter preferably that of the albumin gene.

10. Vector as claimed in one of the claims 2 to 9,.
**wherein**
a viral nuclear establishing sequence such as that of the Epstein-Barr virus or an autonomous replication sequence is used as the establishing sequence.

11. Recombination vector for preparing a recombinant insect virus vector as claimed in one of the claims 1 to 10 comprising a foreign DNA sequence under the control of a promoter suitable for gene expression in the liver and recombination sequences from non-essential regions of the insect virus DNA.

12. Process for producing the vector as claimed in one of the claims 1 to 10,
**wherein**
the foreign DNA sequence together with the promoter is cloned into a recombination vector, the construct obtained is transfected together with the DNA of an insect virus into insect cells and the vector packaged in the insect cells is isolated from the supernatant of the insect cell culture.

13. Process for producing the vector as claimed in 12,
**wherein**
an establishing sequence is inserted before or after cloning.

14. Pharmaceutical composition,
**wherein**
it contains a recombinant insect virus vector as claimed in one of the claims 1 to 10.

15. Use of a recombinant insect virus vector as claimed in one of the claims 1 to 10 or a pharmaceutical composition as claimed in claim 14 to produce an agent for liver-specific gene therapy.

16. Use as claimed in claim 15,
**wherein**
the vector is formulated in such a form that it can be administered to a patient via the blood stream preferably via the portal vein of the liver.

17. Use as claimed in claim 15,
**wherein**
the vector can be administered to a patient via the gastro-intestinal tract.

## Revendications

1. Vecteur convenant pour l'expression génique spécifique du foie consistant en un virus d'insecte qui contient les composants suivants :
- une séquence d'ADN étranger sous le contrôle
- d'un promoteur convenant pour l'expression génique dans des cellules hépatiques.

2. Vecteur selon la revendication 1 **caractérisé en ce qu'**il contient une séquence d'établissement.

3. Vecteur selon la revendication 1 **caractérisé en ce que** l'on utilise une séquence thérapeutique comme séquence d'ADN étranger.

4. Vecteur selon l'une des revendications 1 à 3 **caractérisé en ce que** l'on utilise un baculovirus comme virus d'insecte.

5. Vecteur selon l'une des revendications 1 à 4 **caractérisé en ce que** l'on utilise un virus de polyédrose nucléaire comme virus d'insecte.

6. Vecteur selon les revendications 1 à 5 **caractérisé en ce que** l'on utilise comme séquence d'ADN étranger l'ADNc d'un gène qui est défectueux dans la maladie à traiter.

7. Vecteur selon l'une des revendications 1 à 6 **caractérisé en ce que** l'on utilise comme séquence d'ADN étranger une partie d'une séquence génomique qui recouvre une mutation dans le gène cible et qui peut réaliser une recombinaison homologue avec celle-ci.

8. Vecteur selon l'une des revendications 1 à 7 **caractérisé en ce que** l'on utilise comme séquence d'ADN étranger l'ADNc du récepteur de LDL, de l'alpha-1-antitrypsine, des facteurs de coagulation sanguine VIII et IX, de l'érythropoïétine ou de la thymidine kinase.

9. Vecteur selon l'une des revendications 1 à 8 **caractérisé en ce que** l'on utilise comme promoteur un promoteur viral fort, de préférence le promoteur très précoce du cytomégalovirus (CMV) ou un promoteur spécifique du foie, de préférence celui du virus de l'hépatite B, ou un promoteur de mammifère spécifique du foie, de préférence celui du gène de l'albumine.

10. Vecteur selon l'une des revendications 2 à 9 **caractérisé en ce que** l'on utilise comme séquence d'établissement une séquence d'établissement nucléaire virale comme celle du virus d'Epstein-Barr, ou une séquence de réplication autonome.

11. Vecteur de recombinaison pour la production d'un vecteur de virus d'insecte recombiné selon l'une des revendications 1 à 10 comprenant une séquence d'ADN étranger sous le contrôle d'un promoteur convenant pour l'expression génique dans le foie et des séquences de recombinaison provenant de domaines non essentiels de l'ADN de virus d'insecte.

12. Procédé de production du vecteur selon l'une des revendications 1 à 10 **caractérisé en ce que** la séquence d'ADN étranger est clonée avec le promoteur dans un vecteur de recombinaison, la construction obtenue est utilisée avec l'ADN d'un virus d'insecte pour transfecter des cellules d'insecte et le vecteur empaqueté dans les cellules d'insecte est obtenu à partir du surnageant de la culture de cellules d'insecte.

13. Procédé de production du vecteur selon la revendication 12 **caractérisé en ce qu'**une séquence d'établissement est insérée avant ou après le clonage.

14. Composition pharmaceutique **caractérisée en ce qu'**elle contient un vecteur de virus d'insecte recombiné selon l'une des revendications 1 à 10.

15. Utilisation d'un vecteur de virus d'insecte recombiné selon l'une des revendications 1 à 10 ou d'une composition pharmaceutique selon la revendication 14 pour la production d'un agent pour la thérapie génique spécifique du foie.

16. Utilisation selon la revendication 15 **caractérisée en ce que** le vecteur est confectionné sous une forme telle qu'il peut être administré à un patient par le biais de la circulation sanguine, de préférence par le biais de la veine porte du foie.

17. Utilisation selon la revendication 15 **caractérisée en ce que** le vecteur peut être administré à un patient par le biais des voies gastro-intestinales.
